(19) Europäisches Patentamt
European Patent Office
Office européen des brevets

(11) Veröffentlichungsnummer : **0 071 051**
**B1**

(12) # EUROPÄISCHE PATENTSCHRIFT

(45) Veröffentlichungstag der Patentschrift : **07.05.86**

(51) Int. Cl.⁴ : **C 07 D285/00**, A 01 N 43/72

(21) Anmeldenummer : **82106045.6**

(22) Anmeldetag : **07.07.82**

(54) **3,4,5,6-Tetrahydro-1,2,4,6-thiatriazin(3,5)-dion-1,1-dioxide, Verfahren zu ihrer Herstellung und ihre Verwendung zur Bekämpfung unerwünschten Pflanzenwuchses.**

(30) Priorität : **18.07.81 DE 3128527**

(43) Veröffentlichungstag der Anmeldung : **09.02.83 Patentblatt 83/06**

(45) Bekanntmachung des Hinweises auf die Patenterteilung : **07.05.86 Patentblatt 86/19**

(84) Benannte Vertragsstaaten : **AT BE CH DE FR GB IT LI NL SE**

(56) Entgegenhaltungen :
**EP-A- 0 037 482**
**FR-A- 2 193 826**
**US-A- 3 435 031**
**US-A- 3 817 993**
**R. Wegler "Chemie der Pflanzenschutz- und Schädlingsbekämpfungsmittel" Band 2, Springer-Verlag, Berlin, 1970, S. 359**
***Angewandte Chemie*, Band 93, Seite 157 (1981)**
**R. Wegler "Chemie der Pflanzenschutz- und Schädlingsbekämpfungsmittel" Springer-Verlag, Berlin, 1982, Seiten 97, 125, 196, 332, 333, 357**
**Houben-Weyl "Methoden der Organischen Chemie", 4. Auflage, Band 11, 2, Seite 747 (1958)**
**Chemical Reviews, Band 56, Seite 95 (1956)**

(73) Patentinhaber : **BASF Aktiengesellschaft**
**Carl-Bosch-Strasse 38**
**D-6700 Ludwigshafen (DE)**

(72) Erfinder : **Acker, Rolf-Dieter, Dr.**
**Tuchbleiche 8**
**D-6906 Leimen (DE)**
Erfinder : **Hamprecht, Gerhard, Dr.**
**Rote-Turm-Strasse 28**
**D-6940 Weinheim (DE)**
Erfinder : **Wuerzer, Bruno, Dr. Dipl.-Landwirt**
**Ruedigerstrasse 13**
**D-6701 Otterstadt (DE)**

**EP 0 071 051 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft 3,4,5,6-Tetrahydro-1,2,4,6-thiatriazin(3,5)-dion-1,1-dioxide, Verfahren zu ihrer Herstellung, Herbizide, die diese Verbindungen als Wirkstoffe enthalten, sowie Verfahren zur Bekämpfung unerwünschten Pflanzenwuchses mit diesen Verbindungen.

In der US-PS 3 435 031 sind substituierte 3,4,5,6-Tetrahydro-1,2,4,6-thiatriazin(3,5)-dion-1,1-dioxide als Bakterizide, Fungizide und Fliegen-Repellentien beschrieben. Daß Verbindungen dieser Struktur eine herbizide Wirkung haben, war bisher nicht bekannt. Aus EP-A-0 037 482 sind herbizide 3,4,5,6-Tetrahydro-1,2,4,6-thiatriazin(3,5)-dion-1,1-dioxide bekannt,

Es wurde gefunden, daß 3,4,5,6-Tetrahydro-1,2,4,6-thiatriazin(3,5)-dion-1,1-dioxide der Formel

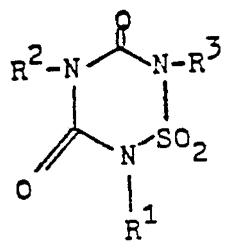

(I),

in der

$R^1$ Wasserstoff, ein Metallatom, einen gegebenenfalls substituierten Ammoniumrest, einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen oder einen gegebenenfalls halogensubstituierten Benzylrest,

$R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen substituierten Phenylrest und

$R^3$ einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen, einen gegebenenfalls halogensubstituierten Benzylrest oder einen durch Halogen, Alkyl, Halogenalkyl, Alkoxy, Halogenalkoxy, Alkylthio, Halogenalkylthio, Halogenalkylsulfonyl mit 1 bis 4 Kohlenstoffatomen substituierten Phenylrest bedeuten, herbizid wirksam sind.

$R^1$ in Formel I bedeutet beispielsweise Alkalimetallatome, wie Natrium, Kalium, oder einen gegebenenfalls durch Alkyl oder Hydroxyalkyl mit jeweils 1 bis 10 Kohlenstoffatomen, vorzugsweise 1 bis 4 Kohlenstoffatomen, oder Cycloalkyl mit 3 bis 7 Kohlenstoffatomen substituierten Ammoniumrest, wie Ammonium, Dimethylammonium, Tridecylammonium, Trimethylammonium, Triethylammonium, Diisopropylethylammonium, Diisopropylmethylammonium, N-Methyl-N,N-diethanolammonium, Cyclohexylammonium.

$R^1$ kann außerdem für einen gesättigten oder ungesättigten unverzweigten aliphatischen Rest mit bis zu 10 Kohlenstoffatomen, vorzugsweise bis zu 4 Kohlenstoffatomen, oder für einen gesättigten oder ungesättigten verzweigten aliphatischen Rest mit 3 bis 10 Kohlenstoffatomen, vorzugsweise bis zu 4 Kohlenstoffatomen, beispielsweise einen Alkylrest, Halogenalkylrest, Alkoxyalkylrest, Alkenylrest, Alkinylrest, Alkylthioalkylrest, Cycloalkoxyalkylrest oder Cycloalkylalkylrest, wie Methyl, Ethyl, Propyl, Isopropyl, n-Butyl, Isobutyl, sec.-Butyl, tert.-Butyl, n-Pentyl, n-Hexyl, 1-Ethyl-n-propyl, 1,2-Dimethyl-n-propyl, 1,3-Dimethyl-n-butyl, 2-Chlorethyl, 2-Chlorpropyl, 3-Chlorpropyl, 2-Chlorisopropyl, 1-Chlormethyln-propyl, 1-Ethyl-2-methyl-n-propyl, 1,2,2-Trimethyl-n-propyl, 1,2-Dimethyl-n-hexyl, 2-Chlor-sec.-butyl, 2-Chlor-isobutyl, 2-Fluor-sec.-butyl, 2-Fluor-isobutyl, 2-Fluorisopropyl, tert.-Amyl, 2-Chlor-tert.-butyl, 2,2,2-Trifluorethyl, Methoxyethyl, Ethoxyethyl, 3-Methoxy-n-propyl, Methoxy-isopropyl, 3-Methoxy-n-butyl, 1-Methoxy-n-propyl, Ethoxy-tert.-butyl, Methoxy-tert.-butyl, 2-Methoxy-n-butyl, 4-Methoxy-n-butyl, Allyl, Methallyl, Crotyl, 2-Ethylhexen-2-yl, Hexen-5-yl, 2-Methyl-buten-2-yl, 2-Methyl-isobut-2-enyl, Isobut-2-inyl-3, Butin-2-yl, Isobut-2-enyl, Propargyl, 2-Methyl-but-3-enyl, 2-Methyl-but-2-enyl, 1-Methyl-isobut-2-enyl, Methylmercapto-ethyl, Ethylmercapto-ethyl, 3-Methylmercapto-n-propyl, 3-Methylmercapto-n-butyl, 1-Methylmercapto-butyl-2, Methylmercapto-tert.-butyl, 2-Methylmercapto-butyl, Cyclohexoxy-ethyl, 1-Cyclohexyl-ethyl, für einen cycloaliphatischen Rest, beispielsweise einen Cycloalkylrest mit 3 bis 7 Kohlenstoffatomen, wie Cyclopropyl, Cyclopentyl, Cyclohexyl, oder für Benzyl oder für einen halogensubstituierten Benzylrest, wobei Halogen Fluor, Chlor, Brom oder Jod sein kann, z. B. 2,6-Dichlorbenzyl, 2,6-Difluorbenzyl, 2-Chlor-6-fluorbenzyl, stehen.

$R^3$ in Formel I bedeutet gesättigte oder ungesättigte unverzweigte aliphatische Reste mit bis zu 10 Kohlenstoffatomen, vorzugsweise bis zu 4 Kohlenstoffatomen, oder gesättigte oder ungesättigte verzweigte aliphatische Reste mit 3 bis 10 Kohlenstoffatomen, vorzugsweise bis zu 4 Kohlenstoffatomen, beispielsweise Alkylreste, Halogenalkylreste, Alkoxyalkylreste, Alkenylreste, Alkinylreste, Alkylthioalkylreste, Cycloalkoxyalkylreste oder Cycloalkylalkylreste, wie Methyl, Ethyl, Propyl, Isopropyl, n-Butyl,

Isobutyl, sec.-Butyl, tert.-Butyl, n-Pentyl, n-Hexyl, 1-Ethyl-n-propyl, 1,2-Dimethyl-n-propyl, 1,3-Dimethyl-n-butyl, vorzugsweise Methyl, 2-Chlorethyl, 2-Chlorpropyl, 3-Chlorpropyl, 2-Chlorisopropyl, 1-Chlormethyln-propyl, 1-Ethyl-2-methyl-n-propyl, 1,2,2-Trimethyl-n-propyl, 1,2-Dimethyl-n-hexyl, 2-Chlor-sec.-butyl, 2-Chlor-isobutyl, 2-Fluor-sec.-butyl, 2-Fluor-isobutyl, 2-Fluorisopropyl, tert.-Amyl, 2-Chlor-tert.-butyl, 2,2,2-Trifluorethyl, Methoxyethyl, Ethoxyethyl, 3-Methoxy-n-propyl, Methoxy-isopropyl, 3-Methoxy-n-butyl, 1-Methoxyethyl-n-propyl, Ethoxy-tert.-butyl, Methoxy-tert.-butyl, 2-Methoxy-n-butyl, 4-Methoxy-n-butyl, Allyl, Methallyl, Crotyl, 2-Ethylhexen-2-yl, Hexen-5-yl, 2-Methyl-buten-2-yl, 2-Methyl-isobut-2-enyl, Isobut-2-inyl-3, Butin-2-yl, Isobut-2-enyl, Propargyl, 2-Methyl-but-3-enyl, 2-Methyl-but-2-enyl, 1-Methyl-isobut-2-enyl, Methylmercapto-ethyl, Ethylmercapto-ethyl, 3-Methylmercapto-n-propyl, 3-Methylmercapto-n-butyl, 1-Methylmercapto-butyl-2, Methylmercapto-tert.-butyl, 2-Methylmercapto-butyl, Cyclohexoxy-ethyl, 1-Cyclohexylethyl, cycloaliphatische Reste, beispielsweise Cycloalkylreste mit 3 bis 7 Kohlenstoffatomen, wie Cyclopropyl, Cyclopentyl, Cyclohexyl, oder Benzyl oder halogensubstituierte Benzylreste, wobei Halogen Fluor, Chlor, Brom oder Jod sein kann, z. B. 2,6-Dichlorbenzyl, 2,6-Difluorbenzyl, 2-Chlor-6-fluorbenzyl.

Darüber hinaus kann $R^3$ Phenyl bedeuten. Weiterhin bedeutet $R^3$, wie auch $R^2$, durch Halogen, Alkyl, Halogenalky, Alkoxy, Halogenalkoxy, Alkylthio, Halogenalkylthio oder Halogenalkylsulfonyl mit jeweils 1 bis 4 Kohlenstoffatomen substituiertes Phenyl, wie o-, m-, p-Chlorphenyl, o-, m-, p-Fluorphenyl, o-, m-, p-Bromphenyl, 2,3-, 2,4-, 2,5-, 2,6-, 3,4-, 3,5-Dichlorphenyl, 2,4,5-Trichlorphenyl, 3,4-Difluorphenyl, 3-Chlor-4-fluorphenyl, 3-Fluor-4-chlorphenyl, o-, m-, p-Methoxy-, o-, m-, p-Ethoxy-, o-, m-, p-Isopropoxy-, o-, m-, p-Trifluormethoxy-, o-, m-, p-Methyl-, o-, m-, p-Ethyl-, o-, m-, p-n-Propyl-, o-, m-, p-Isopropyl-, o-, m-, p-Isobutyl-, o-, m-, p-tert.-Butyl-, o-, m-, p-Methylthio-, o-, m-, p-Trichlormethylthio-, o-, m-, p-Trifluormethylthio-, o-, m-, p-Trifluormethyl-, o-, m-, p-Difluorethyl-, o-, m-, p-Trifluorethyl-, o-, m-, p-Tetrafluorethyl-, o-, m-, p-Pentafluorethyl-, o-, m-, p-Trifluormethylsulfonyl-, 3-Chlor-4-methoxy-, 3-Chlor-4-brom- oder 3-Chlor-4-methylphenyl. Bevorzugte Reste für $R^2$ sind 2-Halogenphenylreste, beispielsweise 2-Fluorphenyl.

Die Verbindungen der Formel I lassen sich herstellen, indem man

a) falls $R^1$ Wasserstoff oder einen gegebenenfalls substituierten Ammoniumrest bedeutet, einen N-Aryl-N'-alkylharnstoff der Formel

$$R^2-NH-\overset{O}{\overset{\|}{C}}-NH-R^3 \qquad (II)$$

in der $R^2$ und $R^3$ die oben genannten Bedeutungen haben, mit Chlorsulfonylisocyanat, gegebenenfalls in Gegenwart eines Säureakzeptors und eines inerten Lösungsmittels bei einer Temperatur im Bereich von — 40 bis + 100 °C oder

b) einen Harnstoff der Formel

$$R^2-NH-\overset{O}{\overset{\|}{C}}-NH-R^3 \qquad (II)$$

oder einen Harnstoff der formel

$$R^2-NH-\overset{O}{\overset{\|}{C}}-NH_2 \qquad (IV)$$

wobei $R^2$ und $R^3$ die oben genannten Bedeutungen haben, mit einem Sulfamoylchlorid der Formel

$$\begin{array}{l} R^1-N-SO_2Cl \\ \quad\;\; | \\ \quad\; COCl \end{array} \qquad (V)$$

in der $R^1$ die oben genannten Bedeutungen, ausgenommen Wasserstoff, ein Metallatom oder einen gegebenenfalls substituierten Ammoniumrest, hat, oder mit einem Sulfamoylchlorid der Formel

$$\begin{array}{l} R^2-N-SO_2Cl \\ \quad\;\; | \\ \quad\; CO_2R^4 \end{array} \qquad (VI)$$

in der $R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen substituierten Phenylrest und $R^4$ Alkyl oder Cycloalkyl mit bis zu 6 Kohlenstoffatomen bedeuten, gegebenenfalls in Gegenwart eines Säureakzeptors und eines inerten Lösungsmittels bei einer Temperatur von — 20 bis + 100 °C oder

c) ein Sulfondiamid der Formel

$$R^1—NH—SO_2—NH—R^3 \qquad (III),$$

in der $R^1$ und $R^3$ die oben genannten Bedeutungen haben, mit einem Carbamoylchlorid der Formel

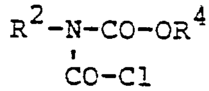

(VII)

in der $R^2$ die oben genannten Bedeutungen hat und $R^4$ Alkyl oder Cycloalkyl mit bis zu 6 Kohlenstoffatomen bedeutet, oder mit einem Carbamoylchlorid der Formel

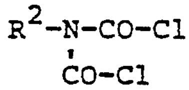

(VIII)

in der $R^2$ die oben genannten Bedeutungen hat, gegebenenfalls in Gegenwart eines Säureakzeptors und eines inerten Lösungsmittels bei einer Temperatur von — 20 °C bis + 100 °C reagieren läßt und die so erhaltenen Verbindungen gegebenenfalls in ihre Salze überführt und/oder, falls $R^1$ und /oder $R^3$ Wasserstoff bedeuten, alkyliert.

Das Verfahren a) läßt sich durch folgendes Formelschema wiedergeben :

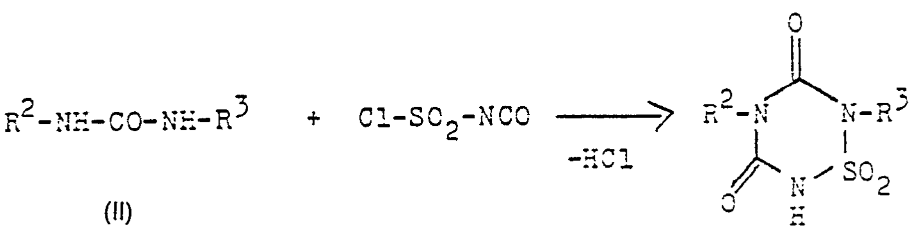


(II) (I)

Die Ausgangsstoffe werden in ungefähr stöchiometrischem Verhältnis eingesetzt, d. h. in einem Unter- bzw. Überschuß von bis zu 20 % Chlorsulfonylisocyanat, bezogen auf den Ausgangsstoff der Formel II. Gegebenenfalls kann ein Säureakzeptor zur Vervollständigung der Reaktion zugesetzt werden.

Überraschend ist der regiospezifisch einheitliche Reaktionsablauf. Trotz der hohen Reaktivität des Chlorsulfonylisocyanats, der Anwesenheit zweier Amidstickstoffe und der bekannten Sulfonierung von Anilinen (J. Chem. Soc. Perkin I, 1043 (1979)) tritt unter den aufgezeigten Reaktionsbedingungen weder eine Sulfonierung des zweiten Stickstoffatoms noch des Arylkerns auf.

Zweckmäßigerweise wird das Verfahren so durchgeführt, daß man das Chlorsulfonylisocyanat bei — 40 °C bis + 100 °C, vorzugsweise — 20 °C bis + 40 °C zu dem Harnstoff der Formel II und einem inerten Lösungsmittel zulaufen läßt. Gegebenenfalls kann der Säureakzeptor nach 0,5 bis 48 Stunden, vorzugsweise 5 bis 28 Stunden bei einer Temperatur von — 20 °C bis + 40 °C in Mengen von 0,5 bis 1,5 Äquivalenten zugegeben werden. Zur Beendigung der Umsetzung rührt-man 5 bis 60 Stunden, vorzugsweise 12 bis 50 Stunden, bei — 20 °C bis + 80 °C nach. Das Reaktionsgemisch wird dann eingeengt. Die gewünschten Endstoffe der Formel I können durch Umfällen, Umkristallieren oder Ausfällen als Salze rein isoliert werden ; gegebenenfalls können sie durch Chromatographie gereinigt werden.

Das Verfahren b) läßt sich-durch folgende Formelschemen wiedergeben :

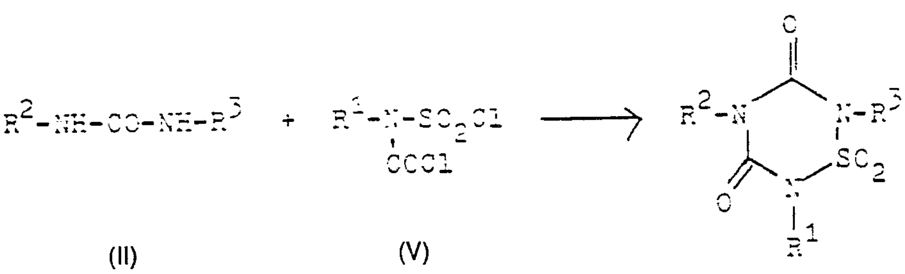

(II) (V) (I)

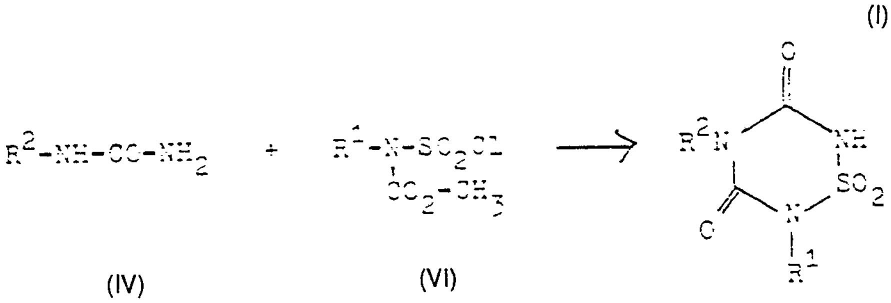

(IV) (VI) (I)

Die Ausgangsstoffe der Formel II und V bzw. IV und VI werden in stöchiometrischem Verhältnis eingesetzt, d. h. in einem Unter- oder Überschuß von bis zu 20 % Ausgangsstoff der Formeln II und IV, bezogen auf V bzw. VI.

Zweckmäßigerweise wird das Verfahren so durchgeführt, daß man über zwei Zuführungen das Carbamoylchlorid der Formeln V bzw. VI und die äquivalente Menge an Säureakzeptor bei einer Temperatur von — 20 °C bis + 100 °C, vorzugsweise bei 0 bis 40 °C, zu einer ungefähr äquivalenten Menge an Harnstoff der Formel IV in einem inerten Lösungsmittel zulaufen läßt. Man rührt anschließend noch 2 bis 24 Stunden bei 0 bis 100 °C, vorzugsweise bei 20 bis 60 °C, nach.

Bei Verwendung von Sulfamoylchloriden der Formel V wird gegebenenfalls eingeengt, bzw. im Falle mit Wasser nicht mischbarer Lösungsmittel mit verd. Salzsäure zur Entfernung der Hydrochloride extrahiert. Die gewünschten Endstoffe der Formel I können dann gegebenenfalls durch Umkristallisieren oder Chromatographie gereinigt werden.

Bei Verwendung von Sulfamoylchloriden der Formel VI kann die Cyclisierung gegebenenfalls nach dem Abtrennen der Hydrochloride in Gegenwart der 1- bis 2,5-fachen Menge Base oder auch im organischen Medium in Gegenwart der 1- bis 2,5-fachen Menge Alkoholat bei einer Temperatur von — 20 °C bis + 100 °C, vorzugsweise von + 20 °C bis + 80 °C, erfolgen. Zur Aufarbeitung der Endstoffe der Formel I säuert man danach an und saugt den anfallenden Niederschlag, gegebenenfalls nach weiterem Einengen, ab. Die gewünschten Endstoffe fallen hierbei in reiner Form an, gegebenenfalls können sie durch Umkristallisieren oder Chromatographie gereinigt werden.

Das Verfahren c) verläuft nach folgenden Schemen :

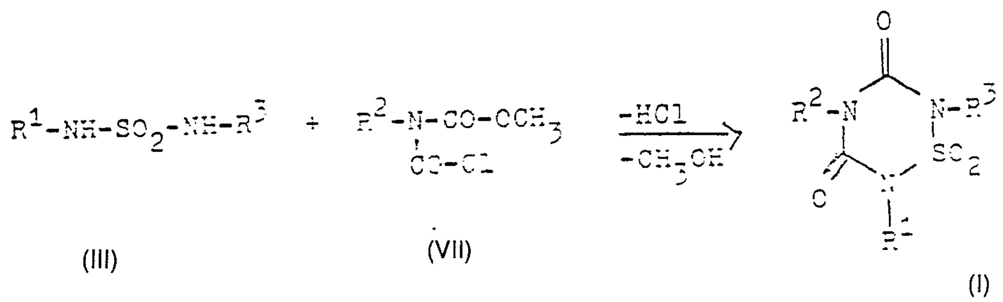


(III) (VII) (I)

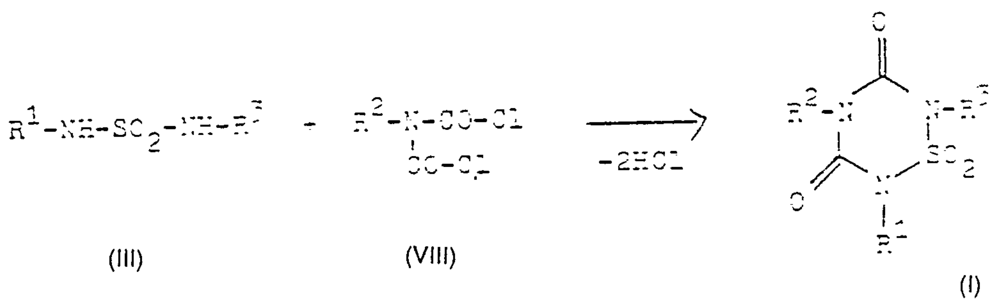


(III) (VIII) (I)

Die Ausgangsstoffe der Formeln III und VII bzw. VIII werden in stöchiometrischem Verhältnis eingesetzt, d. h. in einem Unter- oder Überschuß von bis zu 20 % Ausgangsstoff III, bezogen auf VII bzw. VIII.

Zweckmäßigerweise wird das Verfahren so durchgeführt, daß man über zwei Zuführungen das Carbamoylchlorid der Formeln VII bzw. VIII und die äquivalente Menge an Säureakzeptor bei einer Temperatur von 20 °C bis 100 °C, vorzugsweise bei 0 bis 40 °C, zu einer ungefähr äquivalenten Menge an Sulfondiamid der Formel III in einem inerten Lösungsmittel zulaufen läßt. Zur Beendigung der Umsetzung rührt man noch 2 bis 24 Stunden bei 0 bis 100 °C, vorzugsweise 20 bis 60 °C, nach.

Bei Verwendung von Carbamoylchloriden der Formel VIII wird das Reaktionsgemisch dann gegebenenfalls eingeengt bzw., im Falle mit Wasser nicht mischbarer Lösungsmittel, mit verdünnter Salzsäure zur Entfernung der Hydrochloride extrahiert. Die gewünschten Endstoffe der Formel I können dann gegebenenfalls durch Umkristallisieren oder Chromatographie gereinigt werden.

Bei Verwendung von Carbamoylchloriden der Formel VII kann die Cyclisierung gegebenenfalls nach dem Abtrennen der Hydrochloride in Gegenwart der 1- bis 2,5-fachen Menge Base oder auch im organischen Medium in Gegenwart der 1- bis 2,5-fachen Menge Alkoholat bei einer Temperatur von — 20 °C bis 100 °C, vorzugsweise von + 20 °C bis + 80 °C, erfolgen. Zur Aufarbeitung der Endstoffe der Formel I säuert man danach an und saugt den anfallenden Niederschlag, gegebenenfalls nach weiterem Einengen, ab. Die gewünschten Endstoffe fallen hierbei in reiner Form an, gegebenenfalls können sie durch Umkristallisieren oder Chromatographie gereinigt werden.

Die anschließende Alkylierung der so erhaltenen Verbindungen, in denen $R^1$ Wasserstoff bedeutet, kann mit den entsprechenden Alkylhalogeniden oder mit Dialkylsulfaten erfolgen. Als solche kommen vorzugsweise Methyliodid und Dimethylsulfat in Betracht.

Zweckmäßig wird die Alkylierung so durchgeführt, daß man das Alkylierungsmittel zur Verbindung der Formel I ($R^1$ = H) und gegebenenfalls einem Säureakzeptor in einem inerten Lösungsmittel bei einer Temperatur von 0° bis 100 °C, vorzugsweise 10 °C bis 40 °C zulaufen läßt. Man rührt anschließend noch 2 bis 24 Stunden bei 0 °C bis 100 °C, vorzugsweise bei 20 °C bis 60 °C.

Die Endstoffe der Formel I können gegebenenfalls durch Ausfällen, Einengen der Lösung und Umkristallisieren bzw. durch Chromatographie rein isoliert werden.

Die Verfahren können kontinuierlich oder diskontinuierlich, drucklos oder unter Druck durchgeführt werden, der Einfachheit halber wird bei den Verfahren Atmosphärendruck bevorzugt.

Die Umsetzungen werden in Gegenwart von unter den jeweiligen Reaktionsbedingungen inerten organischen Lösungsmitteln durchgeführt. Als Lösungsmittel kommen beispielsweise Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, m-, p-Dichlorbenzol, o-, m-, p-Chlortoluol, 1,2,4-Trichlorbenzol ; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, 2,2'-Dichlordiethylether ; Nitrokohlenwasserstoffe, wie Nitromethan, Nitroethan, Nitrobenzol, o-, m- p-Chlornitrobenzol, o-Nitrotoluol ; Nitrile, wie Acetonitril, Butyronitril, Isobutyronitril, Benzonitril, m-Chlorbenzonitril ; aliphatische oder cycloaliphatische Kohlenwasserstoffe, z. B. Heptan, Pinan, Nonan, o-, m-, p-Cymol, Benzinfraktionen innerhalb eines Siedepunktintervalls von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan ; Ester, z. B. Ethylacetat, Acetessigester, Isobutylacetat ; Amide, z. B. Formamid, Methylformamid, Dimethylformamid ; Ketone, z. B. Aceton, Methylethylketon ; und entsprechende Gemische. Für das Verfahren a) sind Nitrile als Lösungsmittel zum Erhalt isomerenreiner Verbindungen bevorzugt, insbesondere Acetonitril.

Zweckmäßigerweise verwendet man das Lösungsmittel in einer Menge von 100 bis 2 000 Gew.%, vorzugsweise von 200 bis 700 Gew.%, bezogen auf die Ausgangsstoffe.

Eine anschließende Alkylierung wird zweckmäßigerweise in Wasser oder Alkoholen, z. B. in Methanol, Ethanol, n-Propanol, i-Propanol oder n-Butanol, durchgeführt.

Als Säureakzeptoren können alle üblichen Säurebindemittel verwendet werden. Hierzu gehören vorzugsweise tertiäre Amine, Erdalkalimetallverbindungen, Ammoniumverbindungen und Alkalimetallverbindungen sowie entsprechende Gemische. Es können aber auch Zinkverbindungen verwendet werden. Es kommen z. B. folgende basische Verbindungen in Betracht : Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tri-n-propylamin, Triisopropylamin, Tri-n-butylamin, Triisobutylamin, Tri-sec-butylamin, Tri-tert.-butylamin, Tribenzylamin, Tri-cyclohexylamin, Triamylamin, Diisopropylethylamin,Tri-hexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyrridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Bei Verwendung der Ausgangsstoffe der Formeln VI und VII können gegebenenfalls Stoffe zugesetzt werden, die eine Ringschlußreaktion beschleunigen oder in Gang setzen.

Als Cyclisierungsagentien kommen z. B. die vorgenannten anorganischen Säurebindemittel in Frage, ferner z. B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kaliumtert-butylat, Kaliumethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat.

Das Säurebindemittel wird zweckmäßigerweise in äquivalenten Mengen oder in einem Überschuß von bis zu 20 Gew.%, bezogen auf die Ausgangsstoffe der Formeln VI oder VII, eingesetzt.

Die Ausgangsstoffe der Formeln V, VI, VII, VIII sind bekannt oder lassen sich nach bekannten Methoden herstellen (DE-OS 28 28 969, DE-AS 12 59 871, DE-OS 26 50 014).

Beispiel 1

43,8 Teile N-Methyl-N'-(3',4'-dichlor-phenyl)-harnstoff werden in 130 Teilen Acetonitril vorgelegt. Bei 0 °C werden 28,3 Teile Chlorsulfonylisocyanat portionsweise zugefügt, dann wird 1 Stunde bei 0 °C und 4 Stunden bei Raumtemperatur gerührt. 25,8 Teile Ethyldiisopropylamin werden bei 0 °C zugegeben, man hält 30 Minuten bei 0 °C und rührt 14 Stunden bei Raumtemperatur nach. Die Lösung wird eingeengt, 36 Teile einer 30%igen Natriummethylat-Lösung werden zugegeben. Nach dem Einengen versetzt man mit Essigester bis zur Kristallisation. Man erhält 51 Teile des Ethyldiisopropylammoniumsalzes des 6-Methyl-4-(3',4'-dichlorphenyl)-3,4,5,6-tetrahydro-1,2,4,6-thiatriazin(3,5)-dion-1,1-dioxids (Wirkstoff 1) ; Fp. : 121-123 °C.

Beispiel 2

10 Teile des Wirkstoffs 1 (Beispiel 1) werden in 2 n Natronlauge gelöst. Auf Anreiben fällt nach einiger Zeit ein Niederschlag aus. Nach dem Absaugen und Trocknen ergeben sich 7,1 Teile des Natriumsalzes des 6-Methyl-4-(3',4'-dichlorphenyl)-3,4,5,6-tetrahydro-1,2,4,6-thiatriazin(3,5)-dion-1,1-dioxids (Wirkstoff 2). Fp. : > 240 °C.

Beispiel 3

Zu 15 Teilen des Wirkstoffs 2 (Beispiel 2) wird konzentrierte Salzsäure bis zum Erreichen von pH=0 zugegeben. Die Mischung wird mit Wasser verdünnt und abgesaugt. Man erhält 12 Teile 6-Methyl-4-(3',4'-dichlorphenyl)-3,4,5,6-tetrahydro-1,2,4,6-thiatriazin(3,5)-dion-1,1-dioxid (Wirkstoff 3) von Fp. 170 °C.

Beispiel 4

16,8 Teile N-Methyl-N'-(4-Fluorphenyl)-harnstoff werden in 110 Teilen Acetonitril vorgelegt. Zwischen —10 °C und —5 °C werden 14,2 Teile Chlorsulfonylisocyanat portionsweise zugefügt und 48 Stunden bei Raumtemperatur gerührt. Anschließend werden 10,1 Teile Triethylamin bei —5 °C bis 0 °C zugegeben, man rührt 16 Stunden bei Raumtemperatur nach. Die Lösung wird eingeengt, der Rückstand wird in 70 Teilen Methanol gelöst, dazu werden 18 Teile einer 30-gew.%igen Natriummethylatlösung bei 0 °C bis 5 °C hinzugefügt. Nach 15 Minuten Rühren bei Raumtemperatur wird abfiltriert, und die Lösung wird eingeengt. Man erhält 37 Teile 6-Methyl-4-(4'-fluorphenyl)-3,4,5,6-tetrahydro-1,2,4,6-thiatriazin(3,5)-dion-1,1-dioxid (Wirkstoff 4) als viskoses Öl.

$^1$H-NMR : $\delta$ = 1,20 (t, 3$CH_3$, 9H), 3,12 (q, 3$CH_2$, 6H), 3,10 (s, $CH_3$ 3H), 6,9-7,5 (m, Arom., 4 H)

Beispiel 5

15,5 Teile 6-Methyl-4-(4'-Fluorphenyl)-3,4,5,6-tetrahydro-1,2,4,6-thiatriazin(3,5)-dion-1,1-dioxid (Beispiel 4) werden in 40 Teilen Tetrahydrofuran vorgelegt. 11,8 Teile Methyljodid werden zwischen 20 °C und 25 °C zugetropft. Nach 8 Stunden Rückfluß wird vom Ungelösten filtriert, die Lösung eingeengt und der Rückstand mit Methyl-t-butylether und Wasser kristallisiert. Ausrühren des Niederschlages in Ethanol ergibt 8,1 Teile 2,6-Dimethyl-4-(4'-Fluorphenyl)-3,4,5,6-tetrahydro-1,2,4,6-thiatriazin(3,5)-dion-1,1-dioxid vom Schmp. 133 °C (Zersetzung) (Wirkstoff 5).

Beispiel 6

15,5 Teile des Produktes aus Beispiel 4 werden in 50 Teilen Ethanol gelöst. 7,5 Teile Dimethylsulfat werden zugefügt, die Mischung wird anschließend bei 50 °C 10 Stunden gerührt. Nach Zugabe von 30 Teilen 10 %iger Ammoniaklösung wird mit Methylenchlorid extrahiert, die organische Phase wird getrocknet und eingeengt. Das Anreiben des Rückstandes mit Ethanol ergibt 6,9 Teile des Wirkstoffes 5 (Beispiel 5).

Analog wurden beispielsweise folgende Wirkstoffe der Formel I hergestellt :

(Siehe Tabelle Seite 8 f.)

| Wirkstoff Nr. | $R^1$ | $R^2$ | $R^3$ | Fp. [°C]/$^1H$-NMR |
|---|---|---|---|---|
| 35 | $(C_2H_5)_3N$ | 4-Trifluormethylphenyl | $CH_3$ | 139 (Zers.) |
| 43 | $(C_2H_5)_3N$ | 4-Isopropylphenyl | " | 1,1 - 1,4 (m, 5 $CH_3$), 2,7 - 3,4 (m, 1 CH, 1 $CH_3$, 3 $CH_2$), 6,9 - 7,5 (4 arom. H) |
| 51 | $(C_2H_5)_3N$ | 4-tert.-Butylphenyl | " | 1,0 - 1,5 (m, 6 $CH_3$), 2,9 - 3,5 (m, $CH_3$, 3 $CH_2$), 6,9 - 7,2 (m, 4 aromat.) |
| 73 | $(C_2H_5)_3N$ | 3-Trifluormethylphenyl | " | 1,20 (t, 3 $CH_3$), 3,10 (q, 3 $CH_2$), 3,13 (s, $CH_3$), 7,1 - 8,0 (m, 4 arom. H) |
| 79 | H | 2-Fluorphenyl | $CH_3$ | 174 - 176 |
| 80 | Na | " | " | > 240 |
| 81 | $(C_2H_5)_3N$ | " | " | 102 - 106 |
| 83 | $CH_3$ | " | " | 172 - 175 |
| 98 | $(C_2H_5)_3N$ | 4-Chlorphenyl | " | 137 - 140 |
| 130 | $(C_2H_5)_3N$ | 2,4-Dichlorphenyl | " | 118 - 120 |
| 136 | $(C_2H_5)_3N$ | 3,4-Dichlorphenyl | $CH_3$ | 117 - 120 |
| 137 | $CH_3$ | " | " | 161 - 164 |
| 144 | $(C_2H_5)_3N$ | 4-Fluor-3-chlorphenyl | " | 1,20 (t, 3 $CH_3$), 3,15 (q, 3 $CH_2$), 3,20 (s, $CH_3$, 7,0 - 7,7 (m, 3 arom. H) |
| 152 | $(C_2H_5)_3N$ | 4-Methoxy-3-chlorphenyl | " | 126 (Zers.) |

Analog können beispielsweise folgende Wirkstoffe der Formel I hergestellt werden :

| Wirkstoff Nr. | $R^1$ | $R^2$ | $R^3$ | Fp. [°C]/$^1$H-NMR |
|---|---|---|---|---|
| 17 | H | 4-Methoxyphenyl | $CH_3$ | |
| 18 | Na | 4-Methoxyphenyl | $CH_3$ | |
| 19 | $(C_2H_5)_3N$ | " | " | |
| 20 | $CH_3$ | " | " | |
| 21 | H | " | $i-C_3H_7$ | |
| 22 | Na | " | " | |
| 23 | $(C_2H_5)_3$ | " | " | |
| 24 | $CH_3$ | " | " | |
| 25 | H | 4-Trifluormethoxyphenyl | $CH_3$ | |
| 26 | Na | " | " | |
| 27 | $(C_2H_5)_3N$ | " | " | |
| 28 | $CH_3$ | " | " | |
| 29 | H | " | $i-C_3H_7$ | |
| 30 | Na | 4-Trifluormethoxyphenyl | $i-C_3H_7$ | |
| 31 | $(C_2H_5)_3N$ | " | " | |
| 32 | $CH_3$ | " | " | |
| 33 | H | 4-Trifluormethylphenyl | $CH_3$ | |
| 34 | Na | " | " | |
| 36 | $CH_3$ | " | " | |
| 37 | H | " | $i-C_3H_7$ | |
| 38 | Na | " | " | |
| 39 | $(C_2H_5)_3N$ | " | " | |

(Fortsetzung)

| Wirkstoff Nr. | $R^1$ | $R^2$ | $R^3$ | Fp. [°C]/$^1$H-NMR |
|---|---|---|---|---|
| 40 | $CH_3$ | 4-Trifluormethoxyphenyl | $i\text{-}C_3H_7$ | |
| 41 | H | 4-Isopropylphenyl | $CH_3$ | |
| 42 | Na | " | " | |
| 44 | $CH_3$ | " | " | |
| 45 | H | " | $i\text{-}C_3H_7$ | |
| 46 | Na | " | " | |
| 47 | $(C_2H_5)_3N$ | " | " | |
| 48 | $CH_3$ | " | " | |
| 49 | H | 4-tert.-Butylphenyl | $CH_3$ | |
| 50 | Na | " | " | |
| 52 | $CH_3$ | " | " | |
| 53 | H | " | $i\text{-}C_3H_7$ | |
| 54 | Na | " | " | |
| 55 | $(C_2H_5)_3N$ | " | " | |
| 56 | $CH_3$ | " | " | |
| 57 | H | 4-Fluorphenyl | $CH_3$ | |
| 58 | Na | " | " | |
| 59 | H | " | $i\text{-}C_3H_7$ | |
| 60 | Na | " | " | |
| 61 | $(C_2H_5)_3N$ | " | " | |
| 62 | $CH_3$ | " | " | |
| 63 | H | 3-Fluorphenyl | $CH_3$ | |
| 64 | Na | 3-Fluorphenyl | $CH_3$ | |
| 65 | $(C_2H_5)_3N$ | " | " | |
| 66 | $CH_3$ | " | " | |
| 67 | H | " | $i\text{-}C_3H_7$ | |
| 68 | Na | " | " | |
| 69 | $(C_2H_5)_3N$ | " | " | |

(Fortsetzung)

| Wirkstoff Nr. | $R^1$ | $R^2$ | $R^3$ | Fp. [°C]/$^1H$-NMR |
|---|---|---|---|---|
| 70 | $CH_3$ | " | " | |
| 71 | H | 3-Trifluormethylphenyl | $CH_3$ | |
| 72 | Na | " | " | |
| 74 | $CH_3$ | " | " | |
| 75 | H | " | $i-C_3H_7$ | |
| 76 | Na | " | " | |
| 77 | $(C_2H_5)_3N$ | " | " | |
| 78 | $CH_3$ | " | " | |
| 82 | $(i-C_3H_7)_2C_2H_5N$ | 2-Fluorphenyl | $CH_3$ | |
| 84 | H | " | $C_2H_5$ | |
| 85 | Na | " | " | |
| 86 | $(C_2H_5)_3N$ | " | " | |
| 87 | $CH_3$ | " | " | |
| 88 | H | " | $i-C_3H_7$ | |
| 89 | Na | " | " | |
| 90 | $(C_2H_5)_3N$ | " | " | |
| 91 | $CH_3$ | " | " | |
| 92 | H | " | $C_6H_{11}$ | |
| 93 | Na | " | " | |
| 94 | $(C_2H_5)_3N$ | " | " | |
| 95 | $CH_3$ | 2-Fluorphenyl | $C_6H_{11}$ | |
| 96 | H | 4-Chlorphenyl | $CH_3$ | |
| 97 | Na | " | " | |
| 99 | $CH_3$ | " | " | |
| 100 | H | " | $i-C_3H_7$ | |
| 101 | Na | " | " | |
| 102 | $(C_2H_5)_3N$ | " | " | |
| 103 | $CH_3$ | " | " | |
| 104 | H | 3-Chlorphenyl | $CH_3$ | |
| 105 | Na | " | " | |

(Fortsetzung)

| Wirkstoff Nr. | $R^1$ | $R^2$ | $R^3$ | Fp. [°C]/$^1$H-NMR |
|---|---|---|---|---|
| 106 | $(C_2H_5)_3N$ | " | " | |
| 107 | $CH_3$ | " | " | |
| 108 | H | " | $i-C_3H_7$ | |
| 109 | Na | " | " | |
| 110 | $(C_2H_5)_3N$ | " | " | |
| 111 | $CH_3$ | " | " | |
| 112 | H | 4-Brom-phenyl | $CH_3$ | |
| 113 | Na | " | " | |
| 114 | $(C_2H_5)_3N$ | " | " | |
| 115 | $CH_3$ | " | " | |
| 116 | H | " | $i-C_3H_7$ | |
| 117 | Na | " | " | |
| 118 | $(C_2H_5)_3N$ | " | " | |
| 119 | $CH_3$ | " | " | |
| 120 | H | 3,4-Di-fluor-phenyl | $CH_3$ | |
| 121 | Na | 3,4-Di-fluor-phenyl | $CH_3$ | |
| 122 | $(C_2H_5)_3N$ | " | " | |
| 123 | $CH_3$ | " | " | |
| 124 | H | " | $i-C_3H_7$ | |
| 125 | Na | " | " | |
| 126 | $(C_2H_5)_3N$ | " | " | |
| 127 | $CH_3$ | " | " | |
| 128 | H | 2,4-Di-chlor-phenyl | $CH_3$ | |
| 129 | Na | " | " | |
| 131 | $CH_3$ | " | " | |
| 132 | H | " | $i-C_3H_7$ | |
| 133 | Na | " | " | |
| 134 | $(C_2H_5)_3N$ | " | " | |
| 135 | $CH_3$ | " | " | |

(Fortsetzung)

| Wirkstoff Nr. | $R^1$ | $R^2$ | $R^3$ | Fp. [°C]/$^1$H-NMR |
|---|---|---|---|---|
| 138 | H | 3,4-Dichlorphenyl | $i$-$C_3H_7$ | |
| 139 | Na | " | " | |
| 140 | $(C_2H_5)_3N$ | " | " | |
| 141 | $CH_3$ | " | " | |
| 142 | H | 4-Fluor-3-chlorphenyl | $CH_3$ | |
| 143 | Na | " | " | |
| 145 | $CH_3$ | " | " | |
| 146 | H | " | $i$-$C_3H_7$ | |
| 147 | Na | " | " | |
| 148 | $(C_2H_5)_3N$ | 4-Fluor-3-chlorphenyl | $CH_3$ | |
| 149 | $CH_3$ | " | " | |
| 150 | H | 4-Methoxy-3-chlorphenyl | $CH_3$ | |
| 151 | Na | " | " | |
| 153 | $CH_3$ | " | " | |
| 154 | H | " | $i$-$C_3H_7$ | |
| 155 | Na | " | " | |
| 156 | $(C_2H_5)_3N$ | " | " | |
| 157 | $CH_3$ | " | " | |
| 158 | H | 3-Chlor-4-bromphenyl | $CH_3$ | |
| 159 | Na | " | " | |
| 160 | $(C_2H_5)_3N$ | " | " | |
| 161 | $CH_3$ | " | " | |
| 162 | H | " | $i$-$C_3H_7$ | |
| 163 | Na | 3-Chlor-4-bromphenyl | $i$-$C_3H_7$ | |

(Fortsetzung)

| Wirkstoff Nr. | $R^1$ | $R^2$ | $R^3$ | Fp. [°C]/$^1$H-NMR |
|---|---|---|---|---|
| 164 | $(C_2H_5)_3N$ | " | " | |
| 165 | $CH_3$ | " | " | |
| 166 | H | 4-Methyl-3-chlorphenyl | $CH_3$ | |
| 167 | Na | " | " | |
| 168 | $(C_2H_5)_3N$ | 4-Methyl--3-chlorphenyl | $CH_3$ | |
| 169 | $CH_3$ | " | " | |
| 170 | H | " | $i-C_3H_7$ | |
| 171 | Na | " | " | |
| 172 | $(C_2H_5)_3N$ | " | " | |
| 173 | $CH_3$ | " | " | |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Versteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem fester Trägerstoff hergestellt werden.

Granulate, z. B. Umhüllungs-, Imprägnierungs- und Homogengranulate, können durch Bindung der

Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 83 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 3 Gewichtsteile der Verbindung Nr. 83 werden mit 97 Gewichtsteilen feinteiligem Kaolin vermischt. Man erhält auf diese Weise ein Stäubemittel, das 3 Gewichtsprozent des Wirkstoffs enthält.

V. 30 Gewichtsteile der Verbindung Nr. 81 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VI. 20 Teile der Verbindung Nr. 81 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, vorzugsweise 0,5 bis 5 kg/ha, wobei sich die höheren Dosen besonders zur totalen Bekämpfung von Vegetationen eignen.

Die herbizide Wirkung von Verbindungen der Formel I wird durch Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Kurz vor oder nach dem Aufbringen der Mittel beregnet man die Gefäße leicht, um Keimung und Wachstum in Ganz zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird. Zum Zwecke der Nachauflaufbehandlung zieht man die Testpflanzen je nach Wuchsform erst bis zu einer Wuchshöhe von 3 bis 10 cm an und behandelt sie danach.

Zur Nachauflaufbehandlung werden entweder direkt in die Gefäße gesäte und in den Gefäßen aufgewachsene Pflanzen ausgewählt, oder die Pflanzen werden erst als Keimpflanzen getrennt angezogen und einige Tage vor der Behandlung in die Versuchsgefäße verpflanzt.

Bei den Testpflanzen handelt es sich um Arachys hypogaea (Erdnüsse), Zea mays (Mais) als Vertreter von Kulturpflanzen sowie um die unerwünschten Pflanzen Amaranthus retroflexus (zurückgekrümmter Fuchsschwanz), Chenopodium album (weißer Gänsefuß), Datura stramonium (gemeiner Stechapfel), Sinapis alba (weißer Senf), Ipomoea spp. (Prunkwindenarten) und Solanum nigrum (schwarzer Nachtschatten).

Bei Nachauflaufbehandlung unterbleibt die Abdeckung. Die Versuchsgefäße werden im Gewächshaus aufgestellt, wobei für wärmeliebende Arten wärmere Bereiche (20 bis 35 °C) und für solche gemäßigter Klimate 15 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Ergebnisse dieser Gewächshausversuche zeigt daß die Verbindung Nr. 83 bei Nachauflaufanwendung von 3,0 kg Wirkstoff/ha eine gute herbizide Wirkung zeigt. Die Gewächshausversuche zeigen ferner, daß die Verbindung Nr. 81 bei Vorauflaufanwendung von 2,0 kg Wirkstoff/ha sowie bei Nachauflaufanwendung von 1,0 kg Wirkstoff/ha unerwünschte Pflanzen bekämpft und dabei in selektiver Weise Kulturpflanzen nicht oder nur geringfügig schädigt.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum | |
| (Gossypium arboreum | |
| Gossypium herbaceum | Baumwolle |
| Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |

| Botanischer Name | Deutscher Name |
|---|---|
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht.

Außerdem ist es nützlich, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

**Patentansprüche**

1. 3,4,5,6-Tetrahydro-1,2,4,6-thiatriazin(3,5)-dion-1,1-dioxide der Formel

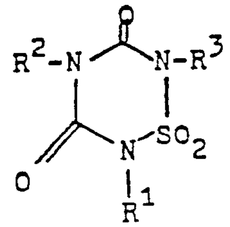

(I)

in der

$R^1$ Wasserstoff, ein Metallatom, einen gegebenenfalls substituierten Ammoniumrest, einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen oder einen gegebenenfalls halogensubstituierten Benzylrest,

$R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen subtituierten Phenylrest und

$R^3$ einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen, einen gegebenenfalls halogensubstituierten Benzylrest oder einen durch Halogen, Alkyl, Halogenalkyl, Alkoxy, Halogenalkoxy, Alkylthio, Halogenalkylthio, Halogenalkylsulfonyl mit 1 bis 4 Kohlenstoffatomen substituierten Phenylrest bedeuten.

2. 3,4,5,6-Tetrahydro-1,2,4,6-thiatriazin(3,5)-dion-1,1-dioxide der Formel I gemäß Anspruch 1, dadurch gekennzeichnet, daß $R^1$ Wasserstoff, unverzweigtes oder verzweigtes Alkyl mit bis zu 7 Kohlenstoffatomen, Cycloalkyl mit 3 bis 7 Kohlenstoffatomen, durch unverzweigtes oder verzweigtes Alkyl mit 1 bis 7 Kohlenstoffatomen oder Cycloalkyl mit 3 bis 7 Kohlenstoffatomen substituiertes Ammonium oder ein Alkalimetallion, $R^2$ einen durch Halogen, Alkyl oder Halogenalkyl mit 1 bis 4 Kohlenstoffatomen substituierten Phenylrest und

$R^3$ unverzweigtes Alkyl mit 1 bis 7 Kohlenstoffatomen oder Cycloalkyl mit 3 bis 7 Kohlenstoffatomen bedeuten.

3. 3,4,5,6-Tetrahydro-1,2,4,6-thiatriazin(3)-dion-1,1-dioxide der Formel I gemäß Anspruch 1, dadurch gekennzeichnet, daß $R^2$ 2-Fluorphenyl bedeutet.

4. Verfahren zur Herstellung von 3,4,5,6-Tetrahydro-1,2,4,6-thiatriazin(3,5)-dion-1,3-dioxiden der Formel I gemäß Anspruch 1, dadurch gekennzeichnet, daß man

a) falls $R^1$ Wasserstoff oder einen gegebenenfalls substituierten Ammoniumrest bedeutet, einen N-Aryl-N'-alkyl-harnstoff der Formel

$$R^2\text{-NH-}\overset{\displaystyle O}{\overset{\|}{C}}\text{-NH-}R^3 \qquad \text{(II)}$$

in der $R^2$ und $R^3$ die im Anspruch 1 genannten Bedeutungen haben, mit Chlorsulfonylisocyanat, gegebenenfalls in Gegenwart eines Säureakzeptors und eines inerten Lösungsmittels bei einer Temperatur im Bereich von —40 bis + 100 °C oder

b) einen Harnstoff der Formel

$$R^2\text{-NH-}\overset{\displaystyle O}{\overset{\|}{C}}\text{-NH-}R^3 \qquad \text{(II)}$$

oder einen Harnstoff der Formel

$$R^2\text{-NH-}\overset{\displaystyle O}{\overset{\|}{C}}\text{-NH}_2 \qquad \text{(IV)}$$

wobei $R^2$ und $R^3$ die im Anspruch 1 genannten Bedeutungen haben, mit einem Sulfamoylchlorid der Formel

$$R^1\text{-}\underset{\displaystyle COCl}{\underset{|}{N}}\text{-}SO_2Cl \qquad \text{(V)}$$

in der $R^1$ die im Anspruch 1 genannten Bedeutungen, ausgenommen Wasserstoff, ein Metallatom oder einen gegebenenfalls substituierten Ammoniumrest, hat, oder mit einem Sulfamoylchlorid der Formel

$$R^2\text{-}\underset{\displaystyle CO_2R^4}{\underset{|}{N}}\text{-}SO_2Cl \qquad \text{(VI)}$$

in der $R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen substituierten Phenylrest und $R^4$ Alkyl oder

Cycloalkyl mit bis zu 6 Kohlenstoffatomen bedeuten, gegebenenfalls in Gegenwart eines Säureakzeptors und eines inerten Lösungsmittels bei einer Temperatur von —20 bis + 100 °C oder

c) ein Sulfondiamid der Formel

$$R^1—NH—SO_2—NH—R^3 \qquad (III),$$

in der $R^1$ und $R^3$ die im Anspruch 1 genannten bedeutungen haben, mit einem Carbamoylchlorid der Formel

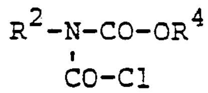
(VII)

in der $R^2$ die im Anspruch 1 genannten Bedeutungen hat und $R^4$ Alkyl oder Cycloalkyl mit bis zu 6 Kohlenstoffatomen bedeutet, oder mit einem Carbamoylchlorid der Formel

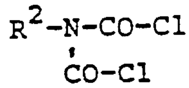
(VIII)

in der $R^2$ die im Anspruch 1 genannten Bedeutungen hat, gegebenenfalls in Gegenwart eines Säureakzeptors und eines inerten Lösungsmittels bei einer Temperatur von —20 °C bis + 100 °C reagieren läßt und die so erhaltenen Verbindungen gegebenenfalls in ihre Salze überführt und/oder, falls $R^1$ Wasserstoff bedeutet, alkyliert.

5. Herbizid, enthaltend ein 3,4,5,6-Tetrahydro-1,2,4,6-thiatriazin(3,5)-dion-1,1-dioxid der Formel

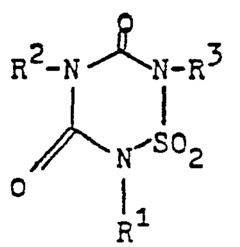
(I)

in der

$R^1$ Wasserstoff, ein Metallatom, einen gegebenenfalls substituierten Ammoniumrest, einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen oder einen gegebenenfalls halogensubstituierten Benzylrest,

$R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen substituierten Phenylrest und

$R^3$ einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen, einen gegebenenfalls halogensubstituierten Benzylrest oder einen durch Halogen, Alkyl, Halogenalkyl, Alkoxy, Halogenalkoxy, Alkylthio, Halogenalkylthio, Halogenalkylsulfonyl mit 1 bis 4 Kohlenstoffatomen substituierten Phenylrest bedeuten.

6. Herbizid, enthaltend inerte Zusatzstoffe und ein 3,4,5,6-Tetrahydro-1,2,4,6-thiatriazin(3,5)-dion-1,1-dioxid der Formel

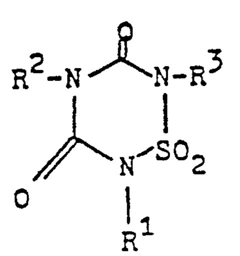
(I)

in der

$R^1$ Wasserstoff, ein Metallatom, einen gegebenenfalls substituierten Ammoniumrest, einen gesättigten oder ungesättigten unverzweigten aliphatischen Rest mit bis zu 10 Kohlenstoffatomen, einen

cycloaliphatischen Rest mit 3 bis 7 Kohlenstoffatomen, einen gesättigten oder ungesättigten verzweigten aliphatischen Rest mit 3 bis 10 Kohlenstoffatomen oder einen gegebenenfalls halogensubstituierten Benzylrest,

$R^2$ einen durch Halogen, Alkyl, Halogenalkyl, Alkoxy, Halogenalkoxy, Alkylthio, Halogenalkylthio oder Halogenalkylsulfonyl mit 1 bis 4 Kohlenstoffatomen substituierten Phenylrest und

$R^3$ einen gesättigten oder ungesättigten unverzweigten aliphatischen Rest mit bis zu 10 Kohlenstoffatomen, einen cycloaliphatischen Rest mit 3 bis 7 Kohlenstoffatomen, einen gesättigten oder ungesättigten verzweigten aliphatischen Rest mit 3 bis 10 Kohlenstoffatomen, einen gegebenenfalls halogensubstituierten Benzylrest oder einen durch Halogen, Alkyl, Halogenalkyl, Alkoxy, Halogenalkoxy, Alkylthio, Halogenalkylthio, Halogenalkylsulfonyl mit 1 bis 4 Kohlenstoffatomen substituierten Phenylrest bedeuten.

7. Herbizid nach Anspruch 6, dadurch gekennzeichnet, daß es ein 3,4,5,6-Tetrahydro-1,2,4,6-thiatriazin(3,5)-dion-1,1-dioxid der Formel I enthält, wobei

$R^1$ Wasserstoff, unverzweigtes oder verzweigtes Alkyl mit bis zu 7 Kohlenstoffatomen, Cycloalkyl mit 3 bis 7 Kohlenstoffatomen, durch unverzweigtes oder verzweigtes Alkyl mit 1 bis 7 Kohlenstoffatomen oder Cycloalkyl mit 3 bis 7 Kohlenstoffatomen substituiertes Ammonium oder ein Alkalimetallion,

$R^2$ einen durch Halogen, Alkyl oder Halogenalkyl mit 1 bis 4 Kohlenstoffatomen substituierten Phenylrest und

$R^3$ unverzweigtes Alkyl mit 1 bis 7 Kohlenstoffatomen oder Cycloalkyl mit 3 bis 7 Kohlenstoffatomen bedeuten.

8. Verfahren zur Bekämpfung unerwünschten Pflanzenwuchses, dadurch gekennzeichnet, daß man die Pflanzen oder den Boden mit einer herbizid wirksamen Menge eines 3,4,5,6-Tetrahydro-1,2,4,6-thiatriazin(3,5)-dion-1,1-dioxids der Formel I gemäß Anspruch 1 behandelt.

## Claims

1. A 3,4,5,6-tetrahydro-1,2,4,6-thiatriazine-3,5-dione 1,1-dioxide of the formula

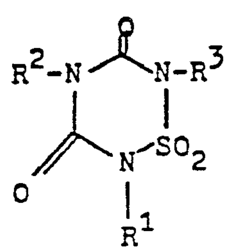

(I)

where

$R^1$ is hydrogen, a metal atom, unsubstituted or substituted ammonium, a saturated or unsaturated straight-chain aliphatic radical of up to 10 carbon atoms, a cycloaliphatic radical of 3 to 7 carbon atoms, a saturated or unsaturated branched aliphatic radical of 3 to 10 carbon atoms, or unsubstituted or halogen-substituted benzyl,

$R^2$ is phenyl which is substituted by halogen, alkyl, haloalkyl, alkoxy, haloalkoxy, alkylthio, haloalkylthio or haloalkylsulfonyl of 1 to 4 carbon atoms, and

$R^3$ is a saturated or unsaturated straight-chain aliphatic radical of up to 10 carbon atoms, a cycloaliphatic radical of 3 to 7 carbon atoms, a saturated or unsaturated branched aliphatic radical of 3 to 10 carbon atoms, unsubstituted or halogen-substituted benzyl, or phenyl which is substituted by halogen, alkyl, haloalkyl, alkoxy, haloalkoxy, alkylthio, haloalkylthio, or haloalkylsulfonyl of 1 to 4 carbon atoms.

2. A 3,4,5,6-tetrahydro-1,2,4,6-thiatriazine-3,5-dione 1,1-dioxide of the formula I as claimed in claim 1, where $R^1$ is hydrogen, straight-chain or branched alkyl of up to 7 carbon atoms, cycloalkyl of 3 to 7 carbon atoms, ammonium substituted by straight-chain or branched alkyl of 1 to 7 carbon atoms or by cycloalkyl of 3 to 7 carbon atoms, or

$R^1$ is an alkali metal ion,

$R^2$ is phenyl which is substituted by halogen, alkyl or haloalkyl of 1 to 4 carbon atoms, and

$R^3$ is straight-chain alkyl of 1 to 7 carbon atoms or cycloalkyl of 3 to 7 carbon atoms.

3. A 3,4,5,6-tetrahydro-1,2,4,6-thiatriazine-3,5-dione 1,1-dioxide of the formula I as claimed in claim 1, where $R^2$ is 2-fluorophenyl.

4. A process for the preparation of 3,4,5,6-tetrahydro-1,2,4,6-thiatriazine-3,5-dione 1,1-dioxide of the formula I as claimed in claim 1, wherein

a) if $R^2$ is hydrogen or unsubstituted or substituted ammonium, an N-aryl-N'-alkylurea of the formula

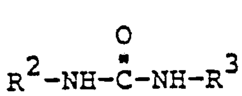

(II)

where $R^2$ and $R^3$ have the meanings given in claim 1, is reacted with chlorosulfonyl isocyanate, in the presence or absence of an acid acceptor and of an inert solvent, at from —40 to + 100 °C, or

b) a urea of the formula

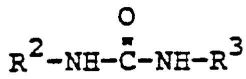

(II)

or of the formula

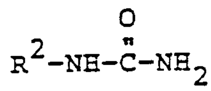

(IV)

where $R^2$ and $R^3$ have the meanings given in claim 1, is reacted with a sulfamyl chloride of the formula

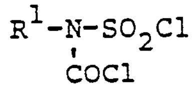

(V)

where $R^1$ has the meanings given in claim 1, with the exception of hydrogen, a metal atom or unsubstituted or substituted ammonium, or with a sulfamyl chloride of the formula

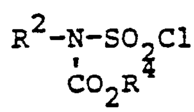

(VI)

where $R^2$ is phenyl which is substituted by halogen, alkyl, haloalkyl, alkoxy, haloalkoxy, alkylthio, haloalkylthio or haloalkylsulfonyl of 1 to 4 carbon atoms, and $R^4$ is alkyl or cycloalkyl of up to 6 carbon atoms, in the presence or absence of an acid acceptor and of an inert solvent, at from — 20 to + 100 °C, or

c) a sulfonyldiamide of the formula

$R^1$—NH—$SO_2$—NH—$R^3$ (III),

where $R^1$ and $R^3$ have the meanings given in claim 1, is reacted with a carbamyl chloride of the formula

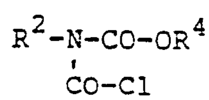

(VII)

where $R^2$ has the meanings given in claim 1, and $R^4$ is alkyl or cycloalkyl of up to 6 carbon atoms, or with a carbamyl chloride of the formula

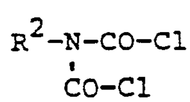

(VIII)

where $R^2$ has the meanings given in claim 1, in the presence or absence of an acid acceptor and of an inert solvent, at from — 20 °C to + 100 °C, and, if desired, the resulting compound may be converted into its salt, and/or, if $R^1$ is hydrogen, alkylated.

5. A herbicide containing a 3,4,5,6-tetrahydro-1,2,4,6-thiatriazine-3,5-dione 1,1-dioxide of the formula

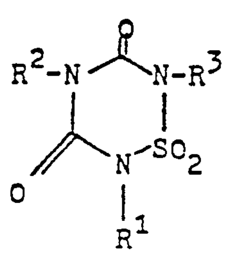

(I)

where

$R^1$ is hydrogen, a metal atom, unsubstituted or substituted ammonium, a saturated or unsaturated straight-chain aliphatic radical of up to 10 carbon atoms, a cycloaliphatic radical of 3 to 7 carbon atoms, a

saturated or unsaturated branched aliphatic radical of 3 to 10 carbon atoms, or unsubstituted or halogen-substituted benzyl,

$R^2$ is phenyl which is substituted by halogen, alkyl, haloalkyl, alkoxy, haloalkoxy, alkylthio, haloalkylthio or haloalkylsulfonyl of 1 to 4 carbon atoms, and

$R^3$ is a saturated or unsaturated straight-chain aliphatic radical of up to 10 carbon atoms, a cycloaliphatic radical of 3 to 7 carbon atoms, a saturated or unsaturated branched aliphatic radical of 3 to 10 carbon atoms, unsubstituted or halogen-substituted benzyl, or phenyl which is substituted by halogen, alkyl, haloalkyl, alkoxy, haloalkoxy, alkylthio, haloalkylthio, or haloalkylsulfonyl of 1 to 4 carbon atoms.

6. A herbicide containing inert additives and a 3,4,5,6-tetrahydro-1,2,4,6-thiatriazine-3,5-dione 1,1-dioxide of the formula

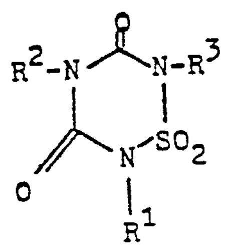

(I)

where

$R^1$ is hydrogen, a metal atom, unsubstituted or substituted ammonium, a saturated or unsaturated straight-chain aliphatic radical of up to 10 carbon atoms, a cycloaliphatic radical of 3 to 7 carbon atoms, a saturated or unsaturated branched aliphatic radical of 3 to 10 carbon atoms, or unsubstituted or halogen-substituted benzyl,

$R^2$ is phenyl which is substituted by halogen, alkyl, haloalkyl, alkoxy, haloalkoxy, alkylthio, haloalkylthio or haloalkylsulfonyl of 1 to 4 carbon atoms, and

$R^3$ is a saturated or unsaturated straight-chain aliphatic radical of up to 10 carbon atoms, a cycloaliphatic radical of 3 to 7 carbon atoms, a saturated or unsaturated branched aliphatic radical of 3 to 10 carbon atoms, unsubstituted or halogen-substituted benzyl, or phenyl which is substituted by halogen, alkyl, haloalkyl, alkoxy, haloalkoxy, alkylthio, haloalkylthio, or haloalkylsulfonyl of 1 to 4 carbon atoms.

7. A herbicide as claimed in claim 6, which contains a 3,4,5,6-tetrahydro-1,2,4,6-thiatriazine-3,5-dione 1,1-dioxide of the formula I as claimed in claim 1, where

$R^1$ is hydrogen, straight-chain or branched alkyl of up to 7 carbon atoms, cycloalkyl of 3 to 7 carbon atoms, ammonium substituted by straight-chain or branched alkyl of 1 to 7 carbon atoms or by cycloalkyl of 3 to 7 carbon atoms, or $R^1$ is an alkali metal ion,

$R^2$ is phenyl which is substituted by halogen, alkyl or haloalkyl of 1 to 4 carbon atoms, and

$R^3$ is straight-chain alkyl of 1 to 7 carbon atoms or cycloalkyl of 3 to 7 carbon atoms.

8. A process for combating the growth of unwanted plants, wherein the plants or the soil are treated with a herbicidally effective amount of a 3,4,5,6-tetrahydro-1,2,4,6-thiatriazine-3,5-dione 1,1-dioxide of the formula I as claimed in claim 1.

**Revendications**

1. 1,1 dioxydes de 3,4,5,6-tétrahydro-1,2,4,6-thiatriazine(3,5)-dione de formule

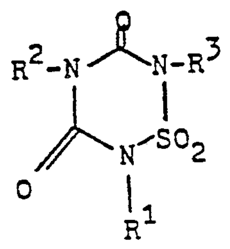

(I)

dans laquelle

$R^1$ représente hydrogène, un atome de métal, un reste d'ammonium éventuellement substitué, un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à 10 atomes de carbone, ou un reste benzyle éventuellement substitué par halogène,

$R^2$ représente un reste phényle substitué par halogène, alkyle, halogénalkyle, alcoxy, halogénalcoxy, alkylthio, halogénalkylthio ou halogénalkylsulfonyle de 1 à 4 atomes de carbone, et

$R^3$ représente un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à

10 atomes de carbone, un reste benzyle éventuellement substitué par halogène ou un reste phényle substitué par halogène, alkyle, halogénalkyle, alcoxy, halogénalcoxy, alkylthio, halogénalkylthio, halogénalkylsulfonyle de 1 à 4 atomes de carbone.

2. 1,1 dioxydes de 3,4,5,6-tétrahydro-1,2,4,6-thiatriazine(3,5)-dione de formule I selon la revendication 1, caractérisés par le fait que

$R^1$ représente hydrogène, alkyle ramifié ou non, ayant jusqu'à 7 atomes de carbone, cycloalkyle de 3 à 7 atomes de carbone, ammonium substitué par alkyle, ramifié ou non, de 1 à 7 atomes de carbone ou cycloalkyle de 3 à 7 atomes de carbone, ou un ion métal alcalin.

$R^2$ représente un reste phényle substitué par halogène, alkyle ou halogénalkyle de 1 à 4 atomes de carbone et

$R^3$ représente alkyle non ramifié de 1 à 7 atomes de carbone ou cycloalkyle de 3 à 7 atomes de carbone.

3. 1,1 dioxydes de 3,4,5,6-tétrahydro-1,2,4,6-thiatriazine(3,5)-dione de formule I selon la revendication 1, caractérisés par le fait que $R^2$ représente 2-fluorophényle.

4. Procédé de préparation de 1,1 dioxyde de 3,4,5,6-tétrahydro-1,2,4,6-thiatriazine (3,5)-dione de formule I selon la revendication 1, caractérisé par le fait que l'on fait réagir

a) dans le cas où $R^1$ représente hydrogène ou un reste ammonium éventuellement substitué, une N-aryl-N'-alkylurée de formule

$$R^2-NH-\overset{O}{\overset{\parallel}{C}}-NH-R^3 \qquad (II)$$

dans laquelle $R^2$ et $R^3$ ont les significations données à la revendication 1, avec du chlorosulfonylisocyanate, éventuellement en présence d'un accepteur d'acide et d'un solvant inerte, à une température comprise dans les limites de — 40 à + 100 °C ou

b) une urée de formule

$$R^2-NH-\overset{O}{\overset{\parallel}{C}}-NH-R^3 \qquad (II)$$

ou une urée de formule

$$R^2-NH-\overset{O}{\overset{\parallel}{C}}-NH_2 \qquad (IV)$$

où $R^2$ et $R^3$ ont les significations données dans la revendication 1, avec un chlorure de sulfamoyle de formule

$$R^1-\underset{COCl}{\underset{|}{N}}-SO_2Cl \qquad (V)$$

dans laquelle $R^1$ a les significations données dans la revendication 1, à l'exception de hydrogène, un atome métal ou un reste ammonium éventuellement substitué, ou avec un chlorure de sulfamoyle de formule

$$R^2-\underset{CO_2R^4}{\underset{|}{N}}-SO_2Cl \qquad (VI)$$

dans laquelle $R^2$ représente un reste phényle substitué par halogène, alkyle, halogénalkyle, alcoxy, halogénalcoxy, alkylthio, halogénalkylthio ou halogénalkylsulfonyle de 1 à 4 atomes de carbone, et $R^4$ représente alkyle ou cycloalkyle ayant jusqu'à 6 atomes de carbone, éventuellement en présence d'un accepteur d'acide et d'un solvant inerte, à une température de — 20 à + 100 °C ou

c) un sulfondiamide de formule

$$R^1—NH—SO_2—NH—R^3 \qquad (III)$$

dans laquelle $R^1$ et $R^3$ ont les significations indiquées dans la revendication 1, avec un chlorure de carbamoyle de formule

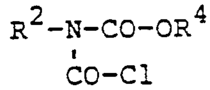

(VII)

dans laquelle $R^2$ a la signification indiquée dans la revendication 1 et $R^4$ représente alkyle ou cycloalkyle ayant jusqu'à 6 atomes de carbone, ou avec un chlorure de carbamoyle de formule

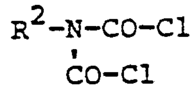

(VIII)

dans laquelle $R^2$ a les significations indiquées dans la revendication 1, éventuellement en présence d'un accepteur d'acide et d'un solvant inerte, à une température de — 20 °C à + 100 °C et on transforme éventuellement en leurs sels les composés ainsi obtenus, et/ou dans le cas où $R^1$ représente hydrogène, on les alkyle.

5. Herbicide contenant un 1,1-dioxyde de 3,4,5,6-tétrahydro-1,2,4,6-thiatriazine(3,5)-dione de formule

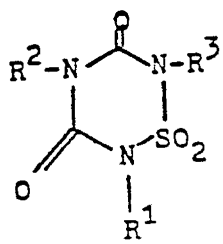

(I)

dans laquelle

$R^1$ représènte hydrogène, un atome de métal, un reste d'ammonium éventuellement substitué, un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à 10 atomes de carbone, ou un reste benzyle éventuellement substitué par halogène,

$R^2$ représente un reste phényle substitué par halogène, alkyle, halogénalkyle, alcoxy, halogénalcoxy, alkylthio, halogénalkylthio ou halogénalkylsulfonyle de 1 à 4 atomes de carbone, et

$R^3$ représente un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à 10 atomes de carbone, un reste benzyle éventuellement substitué par halogène ou un reste phényle substitué par halogène, alkyle, halogénalkyle, alcoxy, halogénalcoxy, alkylthio, halogénalkylthio, halogénalkylsulfonyle de 1 à 4 atomes de carbone.

6. Herbicide contenant des additifs inertes et un 1,1-dioxyde de 3,4,5,6-tétrahydro-1,2,4,6-thiatriazine(3,5)-dione de formule

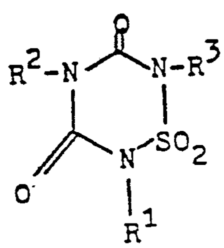

(I)

dans laquelle

$R^1$ représente hydrogène, un atome de métal, un reste d'ammonium éventuellement substitué, un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à 10 atomes de carbone, ou un reste benzyle éventuellement substitué par halogène,

$R^2$ représente un reste phényle substitué par halogène, alkyle, halogénalkyle, alcoxy, halogénalcoxy, alkylthio, halogénalkylthio ou halogénalkylsulfonyle de 1 à 4 atomes de carbone, et

$R^3$ représente un reste aliphatique, non ramifié, saturé ou non, ayant jusqu'à 10 atomes de carbone, un reste cycloaliphatique de 3 à 7 atomes de carbone, un reste aliphatique ramifié, saturé ou non, de 3 à 10 atomes de carbone, un reste benzyle éventuellement substitué par halogène ou un reste phényle substitué par halogène, alkyle, halogénalkyle, alcoxy, halogénalcoxy, alkylthio, halogénalkylthio, halogénalkylsulfonyle de 1 à 4 atomes de carbone.

7. Herbicide selon la revendication 6, caractérisé par le fait qu'il contient un 1,1 dioxyde de 3,4,5,6-tétrahydro-1,2,4,6-thiatriazine(3,5)-dione de formule I, dans laquelle

$R^1$ représente hydrogène, alkyle ramifié ou non, ayant jusqu'à 7 atomes de carbone, cycloalkyle de 3 à 7 atomes de carbone, ammonium substitué par alkyle ramifié ou non, de 1 à 7 atomes de carbone ou cycloalkyle de 3 à 7 atomes de carbone, ou un ion métal alcalin,

$R^2$ représente un reste phényle substitué par halogène, alkyle ou halogénalkyle de 1 à 4 atomes de carbone et

$R^3$ représente alkyle non ramifié de 1 à 7 atomes de carbone ou cycloalkyle de 3 à 7 atomes de carbone.

8. Procédé pour lutter contre la croissance indésirable des plantes caractérisé par le fait que l'on traite les plantes ou le sol avec une quantité efficace, du point de vue herbicide, d'un 1,1 dioxyde de 3,4,5,6-tétrahydro-1,2,4,6-thiatriazine(3,5)-dione de formule I selon la revendication 1.